Europäisches Patentamt

European Patent Office

Office européen des brevets

(11) Publication number: **0 338 716**
**A2**

(12) **EUROPEAN PATENT APPLICATION**

(21) Application number: **89303561.8**

(22) Date of filing: **11.04.89**

(51) Int. Cl.⁴: **C12N 9/64 , C12N 5/00 , C12M 3/00**

(30) Priority: **21.04.88 US 184519**

(43) Date of publication of application:
**25.10.89 Bulletin 89/43**

(84) Designated Contracting States:
**AT BE CH DE ES FR GB GR IT LI LU NL SE**

(71) Applicant: **CODON CORPORATION**
**213 East Grand Avenue**
**South San Francisco California 94025(CA)**

(72) Inventor: **Rice, Craig**
**168 Purcell Drive**
**Alameda California 94501(US)**
Inventor: **King, John Fitch**
**933 Castro Street**
**San Francisco California 94114(US)**

(74) Representative: **Thomson, Paul Anthony et al**
**Potts, Kerr & Co. 15, Hamilton Square**
**Birkenhead Merseyside L41 6BR(GB)**

(54) **Method and system for the production of non-degraded proteins from mammalian cells.**

(57) A method for producing polypeptides said method comprising:perfusing culture media, through a microcarrier bead matrix supporting growth of mammalian cells capable of secreting the polypeptides into the culture media, wherein said mammalian cells are capable of effecting bead-to-bead transfer in the absence of proteolytic enzyme treatment, mechanical disruption, and calcium deprivation; collecting conditioned media from the reactor; and separating the polypeptides from the collected conditioned media.

EP 0 338 716 A2

# METHOD AND SYSTEM FOR THE PRODUCTION OF NON-DEGRADED PROTEINS FROM MAMMALIAN CELLS

## BACKGROUND OF THE INVENTION

### 1. Field of the Invention

The present invention relates generally to methods and systems for the large-scale cultivation of mammalian cell lines. More particularly, it relates to a method and system for the perfusion culture of anchorage-dependent mammalian cells on microcarrier matrices to produce substantially non-degraded protein products.

Many proteins of medical and scientific interest are best produced by the in vitro growth of human and animal cell lines. Unfortunately, the cultivation of such mammalian cell lines is problematic, as most normal diploid cells are anchorage-dependent, requiring attachment to a solid phase support. The requirement of a solid phase support interferes with the ability to rapidly expand a culture within a reactor from an inoculum. Moreover, the high cell concentrations achievable in suspension cultures are much more difficult to attain in attached cell growth reactors.

A variety of methods have been proposed for the large-scale cultivation of anchorage-dependent mammalian cell lines. One of the most promising techniques utilizes a suspension of microcarriers in a reactor vessel to provide the necessary surface area for cell growth. This has proved to be an efficient way to provide a large ratio of surface area to unit reactor volume. Generally, a culture medium is perfused through the vessel, the microcarrier suspension agitated, and product recovered either from the conditioned medium or by detaching and lysing the cells.

Although generally successful, microcarrier perfusion cultures suffer from several drawbacks. Most importantly, it can be very difficult to scale-up or expand the culture from a small inoculum of cells. Frequently, treatment of the microcarrier culture with a proteolytic enzyme is required to detach cells and allow transfer to fresh beads. Such treatment, however, is undesirable as the proteolytic enzyme can degrade protein product which has been secreted into the growth media. While the use of proteolytic enzymes is less problematic if the cells are temporarily removed from the growth media, such a procedure is cumbersome and commercially impractical.

Alternatively, bead-to-bead transfer of cells can be promoted by lowering the divalent cation concentration in the media. Reduced cation concentrations, e.g., calcium ion concentrations, cause suboptimal growth characteristics where the cells are less adherent to the microcarriers. Although generally effective in allowing bead-to-bead transfer, such growth conditions reduce cell viability, lower the production of the desired protein product, and can reduce the quality of homogeneity of the desired polypeptide product.

Finally, with certain cell lines, the cells will bridge between beads allowing initiation of growth on newly-introduced fresh beads. Such bridging, however, can cause aggregation of beads into relatively large clumps, reducing the efficiency with which nutrients are transported to individual cells and with which product is secreted from the cells. Such aggregation can only be prevented with relatively vigorous agitation which can damage the cells, reduce their viability, and have potentially deleterious effects on the polypeptide product.

In addition to the difficulties related to scale-up and expansion, the products obtained from mammalian cell culture are frequently degraded by the mechanical and biochemical conditions present in the reactor vessel. In particular, enzymes which are utilized to promote expansion and which are introduced as a component of serum added to the growth media can degade proteins which are secreted into the conditioned media. The resulting product separated from the conditioned media is often a heterogeneous mix of intact protein and various degradation products. Even after purification, the product will often display reduced activity compared with the intact protein which has been produced under more controlled conditions.

Microcarrier perfusion cultures have also suffered from a limited ability to support high density cell growth and limited cell viability within the reactor. Cell densities have generally not exceeded about 5 to $10 \times 10^6$ cells/ml in most prior perfusion reactors, and continuous reactor runs, i.e., continuous periods in which product may be recovered from the reactor, have generally been limited to 30 days or less.

For the above reasons, it would be desirable to provide methods and systems for the microcarrier perfusion culture of anchorage-dependent mammalian cell lines which facilitate inoculation and cell expansion within a reactor and which result in the production of a superior polypeptide product. Such methods and systems should allow for the bead-to-bead transfer of cells without the need for proteolytic enzyme treatment, cation deprivation, or use of bridging cell cultures. In particular, the methods and systems should provide for cell growth at very high densities without loss of cell viability and without degradation of the proteins due to enzyme activity or other causes. Moreover, the reactor system should create a sterile environment which allows for the long term culturing.

2. Description of the Background Art

Arathoon and Birch (1986) Science 232:1390 and Feder and Tolbert (1983) Scientific American 248:36 review a number of culturing systems for mammalian cell lines, including microcarrier perfusion systems. Microcarrier cell culture systems are specifically described in van Wezel (1967) Nature 216:64; Griffiths et al. (1985) Dev. Biol. Stand. 60:439; and Reuveny (1983) Adv. Biotechnol. Process 2:1. Nahapetian et al. (1986) J. Cell Sci. 81:65, describes a laboratory scale microcarrier perfusion culture system which has been optimized for high density cell growth. Kruithof et al. (1985) Biochem J. 226:631-636 teaches the production of t-PA from a Bowes melanoma cell line grown in tissue culture flats and roller bottles. The product includes substantial amounts of degraded t-PA and appears less able to localize clots than intact t-PA. Kluft et al. (1983) Adv. Biotechnol. Processes 2:97-110 describes the microcarrier culture of a Bowes melanoma cell line producing t-PA. Cell density, however, is limited to about $10^6$ cells/ml, and the product was apparently degraded.

Intact t-PA is a glycoprotein having a molecular weight of about 66,000 daltons, and exists as either a one-chain polypeptide (Bender et al., (1979) J. Biol. Chem. 254:1998-2003) or it may be cleaved by plasmin (Wallen et al. (1981) Prog. in Fibrinolysiss 5:16-23) into a two-chain form, wherein the two polypeptides are linked by a disulfide bond (Rijken et al. (1979) Biochem. Biophys. Acta 580:140-153). Non-glycosylated, enzymaticallly active t-PA has been produced in eukaryotic cells grown in the presence of drugs that prevent glycosylation (Little et al. (1985) Biochemistry 23:6991-6995); and in bacteria (Pennica et al. (1983) Nature 301:214-221). Degraded forms of t-PA, having molecular weights of approximately 50,000 and 32,000 have been found coexisting with intact, one-chain and two-chain t-PA species (Granelli -Piperino & Reich (1978) J. Exp. Med. 148:223-234). Prior art methods for isolating t-PA have not been particularly effective at separating the degraded forms of t-PA from the intact t-PA. In pharmaceutical formulations of t-PA, the availability of substantial quantities of pure intact single-chain enzyme is important and desired. The strong fibrin binding exhibited by t-PA (Thorsen et al. (1972) Throm. Diath. Haemorrh. 28:65-74) is believed to be important for its therapeutic efficacy. The lower molecular weight degraded forms, which have aberrant fibrin binding properties (Banyai et al. (1983) FEBSD Lett. 163:37-41), do not appear to display the specificity and clot localization properties of intact one-chain and two-chain t-PA. Further, it is believed that single-chain t-PA is more desirable in pharmaceutical formulation than the two-chain form due to the much slower rate at which the single-chain form is inactivated by specific inhibitors or t-PA found in plasma (Lecander et al. (1984) Brit. J. Haematol. 57:407-412) and due to the potential systemic activation caused by two-chain t-PA.

## SUMMARY OF THE INVENTION

The present invention provides an improved method and system for the perfusion culture of adherent cell lines on a microcarrier matrix. By employing cell lines capable of bead-to-bead transfer in the absence of proteolytic enzyme treatment, mechanical disruption, and calcium deprivation, the microcarrier perfusion culture can be expanded from a small initial inoculum to a very high production density simply by adding fresh microcarrier particles under controlled conditions of perfusion and agitation to a reactor vessel containing the microcarrier matrix.

The method and system of the present invention have been found to be capable of producing high quality polypeptides which are substantially free from degradation products. The polypeptides are secreted by the cultured mammalian cells into conditioned media, and the desired intact polypeptide can be recovered therefrom with a high degree of homogeneity, typically being at least about 75% of the desired polypeptide with 25% or less degradation products, usually being at least about 90% of the desired polypeptide with 10% or less degradation products, and frequently being 95% or greater of the desired polypeptide with 5% or less degradation products. In the exemplary case, tissue plasminogen activator (t-PA) is recovered from the conditioned media of a reactor run in accordance with the principles of the present invention. The recovered t-PA comprises in excess of 75% by weight of single chain species, preferably in excess of 90% by weight of single chain species, and more preferably in excess of 95% by weight of single chain species. As described in more detail above, the single chain species is generally the preferred product.

The system generally comprises a stirred reactor vessel containing the microcarrier matrix. A cell line capable of synthesizing high levels of a desired product is grown on the microcarrier matrix, and culture media is perfused through the matrix during the production phase. Product is usually secreted into the culture medium by the viable cell line and may be separated from the condition media by conventional techniques. Alternatively, the cells may be periodically harvested to collect materials which are not secreted. The culture media and cell expansion method are adapted to provide the high density perfusion culture of the cell line for extensive periods of time and to allow the desired bead-to-bead transfer of the cells within the matrix.

## BRIEF DESCRIPTION OF THE DRAWINGS

Fig. 1 is a block diagram illustrating the various subsystems of the cell perfusion culture system of the present invention.

Fig. 2 is a schematic illustration of the cell perfusion culture system similar to Fig. 1, but illustrating the internal construction of the reactor vessel in somewhat greater detail.

Fig. 3 shows the increase in cell number of cells inoculated into a 10 liter (working volume) reactor. The arrow indicates a switch from medium containing 5% to 0.5% fetal bovine serum.

Fig. 4 shows the increase in cell number of cells transferred for one reactor into a new reactor. The reactor cultures both prior to and after the transfer were maintained in media supplemented with 0.5% fetal bovine serum.

Fig. 5 shows the daily rate of production of t-PA by culture described in Figure 4.

## DESCRIPTION OF THE SPECIFIC EMBODIMENTS

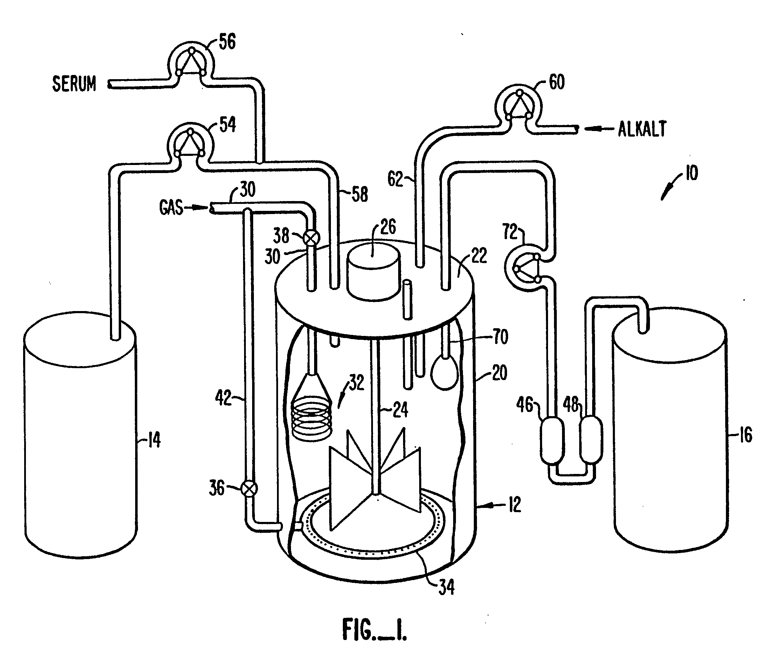

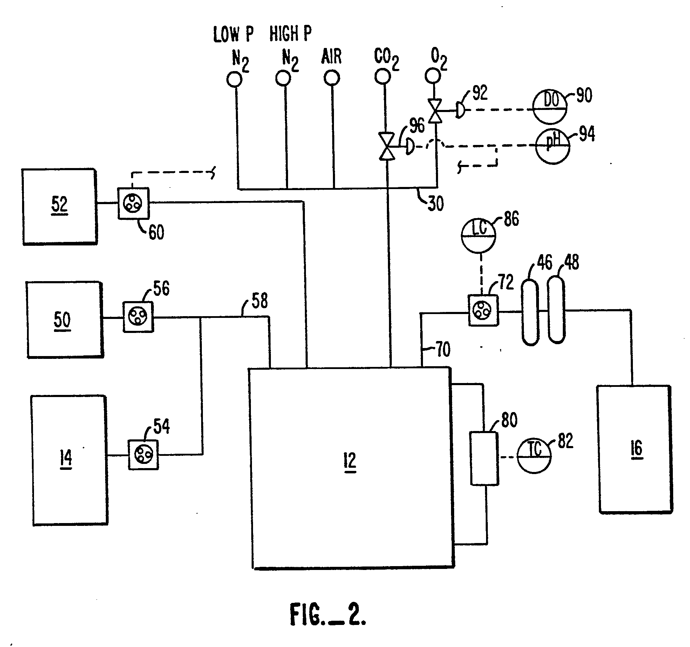

According to the present invention, a system for the large-scale cultivation of an adherent cell line includes a reactor vessel containing a microcarrier matrix upon which the cell line may be grown, a cultural media supply system, a gas supply system, a conditioned media removal system, and several subsystems for controlling temperature, level, pH, dissolved oxygen, and agitation speed within the reactor. The interconnections among the various systems and subsystems are illustrated in Figs. 1 and 2. The method and system of the present invention are preferentially employed to produce tissue plasminogen activator (t-PA) in its preferred single chain form in the conditioned media from the reactor vessel.

### 1. Reactor Vessel

A cell perfusion culture system 10 includes a reactor vessel 12, a culture media tank 14, and a condition media tank 16. The reactor vessel 12 is typically a cylindrical tank 20 which is sealed at its upper end by a head plate 22. The head plate provides a plurality of aseptic penetration ports for the insertion of piping, sensors, and the like. The reactor vessel 12 may be a standard bacterial fermenter of a type which is commercially available. The volume of the reactor vessel will typically be in the range from about 1 to 10,000 liters, usually being in the range from about 10 to 1,000 liters.

Reactor vessel 12 will include an agitator capable of providing low-shear mixing of the vessel contents. Particularly suitable is a large-blade marine impeller 24 which provides both horizontal and vertical mixing at low rotational speeds. Alternatively, a vertically oscillated perforated plate (not illustrated) provides sufficient vertical mixing in improved aeration of the culture media with minimum cell damage. As illustrated, the impeller 24 is driven by an electric motor 26 mounted on the head plate 22.

The reactor vessel 12 will contain microcarriers in a suitable culture media for growing the mammalian cells of interest. The microcarriers are small particles, typically spherical, having dimensions in the range from about 50 to several hundred microns. The microcarriers define a surface suitable for cell attachment

4

and growth and will generally be suspended in the reactor vessel 12 by action of the agitator. In this way, nutrients are delivered to the cells and metabolites removed from the cells in a highly efficient manner while maintaining the cell attachment necessary for growth. The composition of the microcarriers is not critical, and a variety of materials are suitable, including natural polymers, such as dextran, and synthetic polymers, such as methacrylates, styrene, and the like.

The reactor vessel 12 will also include a system for heating and cooling the vessel contents. Conveniently, the heating/cooling system may be a fluid jacket (not illustrated) for receiving a heat exchange medium, described in more detail hereinafter. Alternatively, the heating/cooling system may compise immersed coils (not illustrated) for receiving the heat exchange medium. The design of suitable heating/cooling systems is conventional and need not be described further.

A gas supply manifold 30 (Fig. 2) includes high and low pressure nitrogen connections, as well as sterile air, oxygen, and carbon dioxide connections. Oxygen and carbon dioxide are supplied to the reactor vessel 12 through a gas permeable membrane 32 (Fig. 1) which is immersed within the culture media during operation of the system. Additionally, both the oxygen and carbon dioxide are connected to a sparging ring 34 which is generally at the bottom of the reactor 12. Isolation valves 36 and 38 may select for gas addition through either or both of the gas permeable membrane 32 and sparging ring 34. As will be described in more detail hereinafter, gas introduction will initially be effected through the gas permeable membrane 32 while the culture is being expanded. During the initial stages of expansion, the cells growing on the microcarrier matrix are particularly sensitive to shear damage which can arise as a result of bubbling from the sparger 34. Once high density culture is reached, however, the oxygen demand of the culture increases substantially and the sensitivity to shearing decreases. The gas introduction by sparging becomes desirable at that point in order to provide sufficient oxygen to support the high density culture.

A suitable gas permeable membrane can be constructed from a coil of silicone rubber tubing which is wound around a cylindrical support gauge. Conveniently, the tubing and support gauge are suspended from gas conduit 40 which penetrates through an aseptic port and head plate 22. Sparging ring 34 is supplied by a second gas conduit 42 which branches from the manifold 40 and penetrates through the side wall of the cylindrical tank 20.

## 2. Culture Media Feed System

The culture media feed system includes the culture media tank 14, a serum pre-mix tank 50, and an alkali feed tank 52. The culture media is fed from tank 14 to reactor vessel 12 by a suitable sterile pump 54, typically a peristaltic pump. Similarly, the serum premix is fed from tank 50 through a second sterile pump 56, which will again typically be a peristaltic pump. Conveniently, although not necessarily, the feedline from both the culture media tank 14 and serum premix tank 50 are combined into a single inlet conduit 58 which penetrates the head plate 22 through an aseptic port.

Alkali from tank 52 is transferred by sterile pump 50, again which will usually be a peristaltic pump. The alkali will be fed through a separate inlet conduit 62 which extends through an aseptic port in the head plate 22 and terminates within the tank 20 at a level which will typically be beneath the level of culture media during operation.

## 3. Conditioned Media Recovery System

The conditioned media recovery system includes an elutriation tube 70 which is suspended from head plate 22 and allows the condition media from the reactor vessel 12 to be decanted, while permitting the beads to settle back into the vessel. The construction and operation of elutriation tubes is well known and need not be described further. The elutriation tube 70 is connected to a sterile pump 72, which in turn feeds a vented drip break 46 and a settling chamber 48. The settling chamber 48 should be sized and shaped to provide a minimum flow velocity without unnecessarily increasing the hold-up time. The settling tank 48 allows free-floating cells to settle to the bottom where they can be periodically removed through an aseptic drain line (not illustrated). The conditioned media is collected in conditioned media tank 16 after passing through the settling tank 48.

## 4. System Control

The control system of the present invention may comprise a plurality of discrete automatic controllers or, preferably, a single digital control system which may conveniently be a microprocessor-based control system.

The primary system parameters which are measured and controlled include temperature, level (or volume), pH, and dissolved oxygen of the culture media within the reactor 12. Suitable sensors (not illustrated) will be provided for each of these parameters, typically by inserting a sensor probe through an aseptic port in the head plate 22. Numerous sensors suitable for majoring each of these parameters are commercially available which may be easily adapted to the system of the present invention. The outputs of the sensors will be fed to the control system which will then effect adjustments in the parameter (as described below) based on normal feedback control algorithms.

Secondary system variables which are controlled include the flow rates of culture media from tank 14 and serum premix from tank 50 into reactor 12 (which are conveniently controlled by adjusting the speeds of pumps 54 and 56, respectively), the agitator 24 speed, the oxygen pressure within the membrane 32, the pressure within the reactor head (i.e., the volume above the liquid media surface), the precise oxygen supply composition, microcarrier addition rate, and growth media perfusion rate. The control of the secondary variables will generally not be based on feedback from measured parameters, but rather will be based on the observed cell growth characteristics within the vessel. As will be described in more detail hereinafter, the serum will be added at a higher concentration during the initial stages of operation when the cell culture is being expanded. Similarly, the feed rate of culture media will be controlled by the operator based on a number of observed operating parameters of the system.

Temperature control is achieved by a heater/chiller unit 80 which circulates a heat exchange medium, typically water, through a fluid jacket or other suitable heat exchanger on reactor vessel 12. The temperature and/or flow rate of the heat exchange medium is controlled by temperature controller 82 to maintain a substantially constant temperature within the reactor 12.

Level of the condition media within reactor 12 is controlled by level controller 86 which adjusts the speed of outlet pump 72 which, of course, adjusts the volume rate at which the condition media is drawn from reactor 12. Thus, any changes in the inlet flow of culture medium caused by changes in the throughputs of pump 54 and/or 56 (as selected by the operator) will be automatically compensated for by the level controller 86.

Dissolved oxygen is controlled (usually to a level of about 50% $CO_2$) by a dissolved oxygen controller 90 which adjusts a control valve 92 which modulates the flow rate of oxygen in through the gas permeable membrane 32 and sparging ring 34. When the maximum flow capability of the sparging ring 34 is insufficient to increase the dissolved oxygen concentration to the desired level, flow through the membrane 32 will be commenced.

The pH control is effected by pH controller 94 which adjusts a control valve 96 and pump 60. The control valve 96, in turn, adjusts the inlet flow rate of carbon dioxide, while pump 60 controls the inlet flow rate of alkali 52.

Daily glucose assays will be taken with a commercially available glucose meter. The perfusion rate will be increased by a fixed amount, usually about 0.5 culture volumes/day so long as the glucose concentration remains below a desired level, typically about 1.5 mg/ml. The maximum perfusion rate will be about 2 culture volumes/day.

In addition to the glucose assays, sterility tests, cell counts, cell viability tests, and microscopic examination of the cells will be performed at least once a day for each reactor. The volume of culture media available in tank 14 and remaining capacity of conditioned media tank 16 should also be checked periodically to assure the continuous operation of the system.

## 5. Culture Media

The culture media comprises a base media suitable for mammalian cell growth, such as McCoy's 5A medium. For the inoculation growth phase, the base media will usually be supplemented with a serum source, typically fetal bovine serum (FBS), present at a concentration in the range from about 1 to 10% by weight, usually being present at about 2 to 5% by weight. During the perfusion growth phase, the FBS concentration is usually maintained at a lower concentration, typically being in the range from about 0.1 to 1%, usually being about 0.5%. During both growth phases, the serum source should be treated to remove proteolytic and other enzymes, for example by contacting the serum with lysine-Sepharose as described in co-pending, commonly assigned USSN 167,061, filed on March 11, 1988, the disclosure of which is incorporated herein by reference. For the production of t-PA, it is particularly important to remove plasminogen, as described in USSN 167,061. The use of such "scrubbed" serum helps minimize degradation of the t-PA secreted into the conditioned media and further effects the removal of serum proteins which would otherwise co-purify with the t-PA. Other growth factors may also be added, such as glutamine (optimally at 400 mg/L). Aprotinin (usually at 0.1 to 10 kIU/ml and preferably at 1 to 5 kIU/ml) may be added as a protease inhibitor to further protect the product released into the conditioned media.

## 6. Cell Lines

Cell lines suitable for use in the present invention include mammalian cell lines capable of adherent growth on microcarrier beads and bead-to-bead transfer in the absence of proteolytic enzymes, calcium deprivation, and mechanical disruption. Usually, the cell lines will also be capable of growth in suspension cultures to facilitate propagation of the initial microcarrier inoculum.

Particular cell lines which meet these requirements are Chinese hampster ovaries (CHO) and human melanoma cell lines.

A particularly preferred melanoma cell line is CHL-1 which is described in USSN 167,061, previously incorporated herein by reference. A deposit of the CHL-1 cell has been made at the American Type Culture Collection, 12301 Parklawn Drive, Rockville, Maryland 20852, which deposit has been granted ATCC Accession No. CRL 9446. For the production of t-PA, the CHL-1 cell line may be modified by the introduction of additional copies of the t-PA gene, as described in USSN 167,061.

## 7. Start-Up

Prior to operation, all components of the reactor system 10 will be sterilized, typically by autoclaving. Conveniently, lines to and from the reactor vessel 12 will be covered with narrow pore (0.2 $\mu$m) hydrophobic filters which will allow steam penetration without allowing subsequent entry of microorganisms. The reactor should be autoclaved with liquid covering the various sensor probes, and a vacuum should be drawn on the reactor to prevent air pocket entrapment which can interfere with steam penetration.

The liquid in the vessl 12 is removed to the extent possible through a sample line (not illustrated) and fresh culture media from vessel 14 is provided. A desired amount of the serum premix is also added and an anti-foam controller (not illustrated) is started. The reactor is allowed to agitate for one or two days at 37° C in 100% dissolved oxygen as a sterility test. If the culture medium remains sterile at the termination of the test, it is ready for inoculation.

The reactor vessel 12 may be inoculated by either of two procedures, the first employing cells attached to microcarriers and the second employing cells in suspension. In both cases, the inoculum is expanded from a master working cell bank of frozen aliquots, according to standard cell culture techniques. Once a sufficiently large population is obtained, the reactor vessel 12 may be inoculated.

Using the microcarrier inoculation procedure, a spinner culture of microcarrier particles is allowed to grow to a density of about $1 \times 10^6$ cells/ml. The amount of inoculation culture required will vary depending on the volume of the reactor. Typically, the ratio of inoculum volume to reactor volume will be in the range from about 1:10 to 1:20. Care must be taken to assure that transfer of the spinner culture does not introduce contaminating microorganisms into the reactor vessel 12. Typically, microcarriers are transferred by pressurizing the spinner culture vessel while supplying agitation to keep the microcarriers in suspension. The inoculum is then transferred through a transfer tube by over-pressure to the reactor.

To utilize a suspension inoculum, reactor vessel 12 is filled with a calcium-free growth media. A suspension of cells is obtained by trypsinization from roller bottles and transfer is achieved using a sterile aspirator flask by over-pressure. Cells are transferred to the reactor at a final reactor concentration in the range from about $10^5$ to $10^6$ cells/ml.

## 8. Expansion of the Culture to High Density

After inoculation with either the microcarrier or free-cell suspension, the cell culture will be expanded to production density, typically in the range from about $10^6$ to $3 \times 10^7$ cells/ml. In the case of microcarrier inoculation, the culture is allowed to grow on the initial charge of microcarriers without the addition of fresh media, until the cell density reaches a predetermined intermediate level, typically in the range from about 1 to $2 \times 10^6$ cells/ml or until the glucose residual in the culture media decreases to less than about 25% of its initial level. In the case of a free-cell suspension inoculum, free cell density is allowed to increase without addition of fresh culture media until the cell density reaches about $10^6$ cells/ml. After that density is reached, sufficient calcium is added to the culture medium to render the cells adherent and microcarriers are added, typically to a concentration of about 1 gram of beads per liter of culture medium. In a short time, typically about 24 hrs., the cells attach to the beads, and the remaining expansion procedure is identical for both microcarrier and free cell suspension inoculums.

After the desired cell density on microcarriers is achieved, perfusion of fresh media supplemented with serum premix is initiated. Typically, the concentration of serum in the fresh media will be in the range from about 2% to 10% by weight, more typically in the range from about 3% to 8% by weight, and normally being about 5% by weight. Initially, the perfusion rate will be in the range from about 0.25 to 0.75 culture volumes/day, typically being about 0.5 culture volumes/day. As the cell growth increases, the perfusion rate is increased to a final rate in the range from about 1.5 to 2.5 culture volumes/day, typically over a period of about 2 to 10 days. During the expansion, sterile, pre-equilibrated microcarriers are added to the reactor to maintain the microcarrier to cell density ratio in the range from about 0.5 to 1.0 grams of beads to $10^9$ cells. Conveniently, the beads are added to the reactor using an aspirator through the sample line.

## 9. Production Phase

After cell density has reached the production level, the serum addition to the fresh culture medium will be reduced, typically to a concentration in the range from about 0.1 to 0.5 weight percent. Typically, for the production of t-PA, a scrubbed serum free from plasminogen will be utilized, as described in co-pending application serial no. 076,682.

The culture in production phase requires little attention. Additional culture media, serum, and alkali need to be provided as the supply tanks are depleted. Samples of the condition media should be analyzed at least once a day to assure that production continues free from contamination.

## 10. Batch Production

As an alternative to the continuous production protocol described above, the conditioned medium may be produced by a batch or semi-continuous procedure where the agitator in the reactor vessel is periodically stopped, and the microcarrier beads allowed to settle to the bottom of the reactor. The culture supernatant is rapidly pumped out, typically through the sample line or by adjusting the position of the elutriation tube. Pre-heated fresh media is then pumped back into the reactor in an amount sufficient to restore the operating level. The culture may then be continued, either with or without perfusion, until the next batch of media is withdrawn.

With the method just described, substantially all of the cell culture can be maintained in the reactor in a viable state even while withdrawing most of the culture media. Production of the desired polypeptide is then reinitiated by adding the fresh media. Generally, the batch production method will not be preferred over the continuous production method.

The following examples are offered by way of illustration, not by way of limitation.

## Example 1: Inoculating a Bioreactor Cell Using a Cell Pyramid

An inoculum for a bioreactor is prepared by thawing a frozen ampule containing $10^7$ cells (cell line: W8A5-1 CW) and inoculating these cells into a T-175 flask containing approximately 75 ml of a basal medium such as alpha-MEM or McCoy's Modified 5A medium supplemented with 5% fetal bovine serum. These cells are incubated at 37°C under a humidified atmosphere supplemented to 5% in $CO_2$. After 24 hours, these cells are recovered by trypsinization and propagated into additional tissue culture flasks containing serum supplemented media (described above). Typically 2 to 5 million cells are planted into each flask. After approximately four days, a confluent monolayer is formed by the cells in the flasks, and these cells, typically 10 to 20 million, are recovered by trypsinization. If necessary, these cells are propagated by further passage in T-175's until at least 100 million cells are obtained.

These cells are used to inoculate one or more one liter spinner flasks (Bellco Model 1965) containing 1 gram of Cytodex 3™ (Pharmacia) microcarriers and one liter of McCoy's medium supplemented with 5% fetal bovine serum. The culture is incubated at 37°C under a humidified atmosphere containing 5% $CO_2$

and stirred at 20-30 rpm. When the culture reaches a density of approximately $5 \times 10^5$ cells/ml, typically after 3-4 days, one half of the medium is replaced with fresh growth medium. The culture reaches a density of $10^6$ cells/ml within 1-2 days. The cells and microcarriers in this spinner are aliquoted into two to four spinners, each containing 1 gram of Cytodex 3 beads and 1 liter of medium (described above). These cultures are maintained as described above. When a total of $3 - 4 \times 10^9$ cells have been obtained, the fermentor may be inoculated.

A 15 liter (10 liter working volume) fermentor, purchased from Applikon Dependable Instruments, is configured as shown in Figures 1 and 2 for long-term continuous perfusion. The primary monitoring and controlling instruments were purchased from B. Braun and Athena Controls. This reactor contains up to 6 liters of medium, (McCoy's 5A supplemented with 5% serum, 5 kIU/ml aprotinin and 50 ug/ml gentamicin (optional)), and an additional 1 gram/liter of microcarriers - Cytodex 3. The cultures from the spinners are aseptically transferred into the reactor.

The reactors used are configured to allow for long-term perfusion culture under controlled conditions as described above and shown in Figures 1 and 2. Continuously monitored and controlled are pH, temperature, dissolved oxygen, agitation rate, perfusion rate and reactor volume. The control parameters are summarized below. These are the normal and preferred reactor ranges, however, excursions outside these limits may not be detrimental to the cells or process.

| | |
|---|---|
| pH: | $7.1 \pm 0.2$ |
| Temperature: | $36.5 \pm 0.5$ C |
| Dissolved oxygen: | $50 \pm 10\%$ of air saturation |
| Agitation rate: | 15-50 RPM |
| Reactor volume: | 10 liters |
| Perfusion rate: | 0-3 culture volumes/day |
| Glucose: | 1.25 to 2.0 mg/ml |
| Microcarrier/cell: | approximately 1 gm of Cytodex 3 per billion cells up to a total reactor concentration of 10 to 20 grams/liter. |

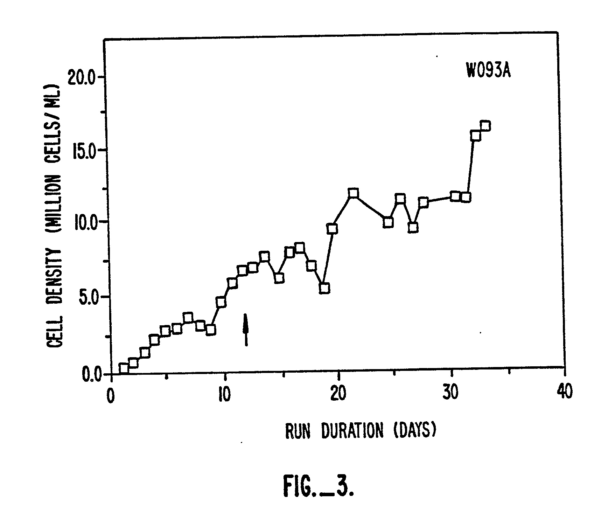

On a daily basis, reactor samples are aseptically removed to permit determinations using standard procedures of cell density, residual glucose, pH and t-PA concentration, and to allow the gross appearance of the cells to be observed. The cell and glucose analyses serve as control criteria for addition of microcarriers, changes of media from growth to production, and changes in perfusion rate. The reactor perfusion rate is grossly varied to maintain the glucose concentration between the aforementioned values. However, there is not a strict adherence between the glucose concentration and the true cell limiting factors in the reactor medium. As warranted, i.e., when the ratio of cells to beads exceed $10^9$ per gram, additional microcarriers are aseptically added to the reactor. Initially, the reactor is perfused with a medium supplemented with 5% fetal bovine serum. The cells are allowed to grow to a density of approximately 2 - $5 \times 10^6$ cells per milliliter (Figure 3), wherein the perfusion medium is replaced with one supplemented McCoy's 5A supplemented with not more than 0.5% serum, 5 kIU/ml aprotinin and gentamicin (optional) - so called "production medium."

The serum used has been pretreated to remove plasminogen and plasmin (see commonly assigned USSN 167,061). The cells continue to grow to densities greater than 10 million cells/ml, and are maintained in a viable and product producing state by continual perfusion with production medium. Reactor samples are regularly drawn and analyzed and the reactor parameters are maintained as decribed above. Once sufficient growth medium has been diluted from the reactor, the effluent stream from the reactor is collected and the product, t-PA, is recovered.

Example 2: Inoculating a Bioreactor with Cells from another Bioreactor

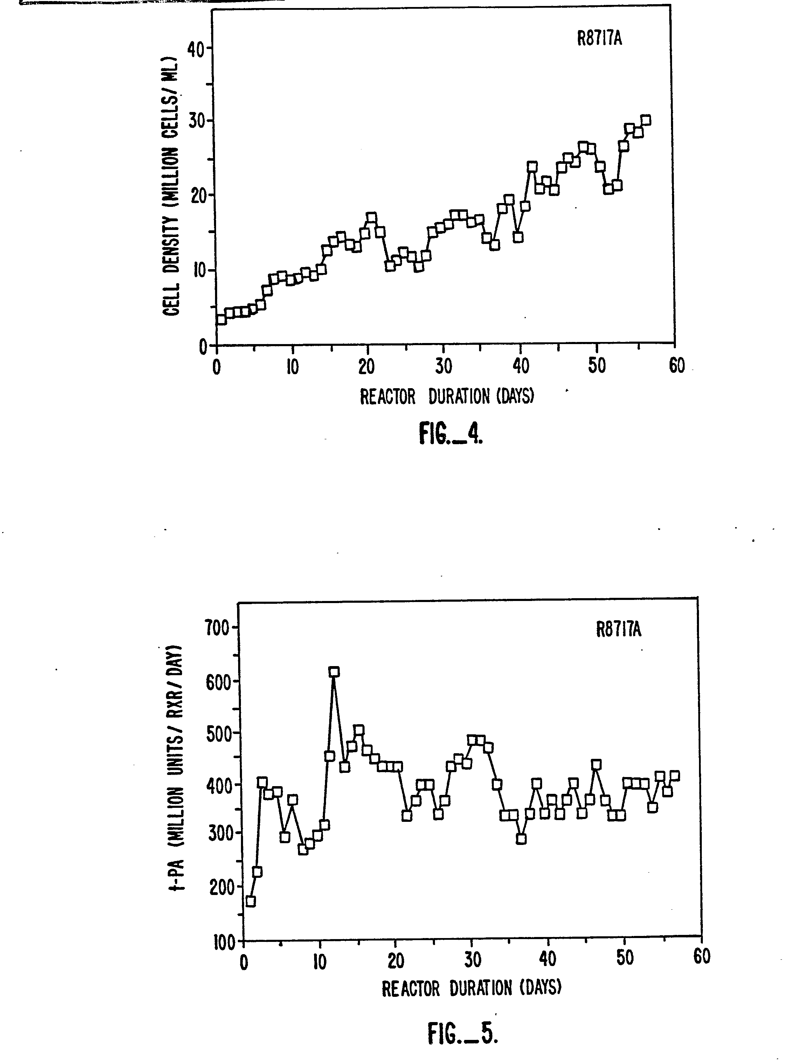

Cells of cell line W8A5-1 were grown and maintained in a bioreactor as described in Example 1 to achieve a culture with a density of 10 million cells/ml maintained in production medium. Approximately 3 liters of these cells were aseptically transferred into a bioreactor, configured as described above, containing approximately 7 liters of fresh production medium (described above). The new reactor was monitored and maintained as described above. The cells transferred into this reactor, after a 1-2 day lag period, resumed growth in the production medium (Figure 4). The culture was maintained in this reactor and stably produced

approximately 400x10$^6$ units of t-pA per day for sixty days (Figure 5).

Example 3: Purification of t-PA from Conditioned Liquid Medium using Urea

Harvest obtained from reactors such as those described in Examples 1 and 2 was processed as follows.

Liquid medium containing 0.5% fetal bovine serum, which had been pre-adsorbed with lysine-Sepharose, and 10 kIU aprotinin per ml was conditioned by incubation with RPMI 7932 cells, or alternatively, other plasminogen activator producing cells.

This conditioned liquid medium was clarified from cells and cellular debris by microfiltration using a Prostak unit with a 0.45 micron filter.

Clarified medium is passed through a 0.2 micron filter, adjusted to approximately pH 7.2 to 7.4 with NaOH, chilled to 4°C, and passed through a chelating Sepharose column complexed with Zn$^{++}$ as recommended by the manufacturer (Pharmacia, Inc.). In a typical chromatographic run, 600 liters of clarified media containing t-PA at a concentration of 10 mg/ml are introduced onto 3.5 l of resin. The column was previously equilibrated with phosphate buffered saline. Up to 200 equivalent column volumes of medium are passed through the resin at rates up to 50 cm per hour for a 10 cm bed of Sepharose CL-6B or 300 cm per hour for an equivalent column of Sepharose FF. Greater than 95% of the t-PA activity will bind to the resins. The column is next washed at a rate of 6 liters per hour with probes monitoring pH, conductivity and optical density. The column is first washed with 20 mM Tris-HCl pH 8.0, 1.0 M NaCl, 0.01% Tween 80, 10 kIU aprotinin per ml followed by 20 mM Tris-HCl (pH 8.0), 25 mM NaCl. 0.01% Tween 80, 10 kIU aprotinin per ml. The column was then washed with the previous buffer plus 0.1 M imidazole which elutes off the degraded forms of t-PA, about 5% of the intact t-PA and most of the non-t-PA protein bound to the column. The remaining intact enzyme is recovered by passing 10 mM Tris-HCl (pH 8.0), 0.5 M NaCl, 50 mM Na EDTA, 0.01% Tween 80, 2.0 M urea and 10 kIU aprotinin/ml at 4°C through the resin. The column effluent is collected. This fraction is typically 50-60% pure.

The partially purified product is next diluted 5-fold with a buffer consisting of 10 mM citrate, 0.05% Tween 80, and 2 M urea at pH 4.7 and the final mixture is adjusted to pH 4.85. This material is pumped through a DEAE resin which has been pre-equilibrated with 0.01% Tween 80, 0.1 M NaCl, 10 mM sodium citrate and 2 M urea at pH 5.0. The DEAE effluent is adjusted to pH 8.0 with NaOH and ethylene glycol is added to a final concentration of 10%.

The resultant pre-lysine t-PA solution (approximately 30 liters) is loaded onto a 2 liter lysine column which has been pre-equilibrated with a buffer of 10 mM Tris, 0.1 M NaCl, 0.01% Tween 80, 2 M urea, pH 7.6 at 4°C. The lysine resin of choice has an extended arm and is derived from coupling of lysine to activated CH-Sepharose 4B.

For elution, the column is washed with 10 mM Tris, 0.5 M NaCl, 0.05% Tween 80, 10% ethylene glycol (100 gm in 1 L), pH 8.0 at 4°C, followed by 10 mM Tris, 0.1 M NaCl, pH 7.6 at 4°C. The t-PA is eluted with 100 mM glycine-HCl, pH 3.0 at 4°C.

The t-PA can be ultrafiltered and concentrated to 20 mg/ml of t-PA in 30 mM glycine-HCl at pH 3.0. Optionally, the t-PA preparation can be passed through an exclusion column such as G-100 prior to sterile filtration.

Although the foregoing invention has been described in some detail by way of illustration and example for purposes of clarity of understanding, it will be obvious that certain changes and modifications may be practiced within the scope of the appended claims.

**Claims**

1. A method for producing polypeptides said method comprising:
perfusing culture media, through a microcarrier bead matrix supporting growth of mammalian cells capable of secreting the polypeptides into the culture media, wherein said mammalian cells are capable of effecting bead-to-bead transfer in the absence of proteolytic enzyme treatment, mechanical disruption, and calcium deprivation;
collecting conditioned media from the reactor; and
separating the polypeptides from the collected conditioned media.

2. A method for producing polypeptides, said method comprising:

a) recovering at least about 50% of conditioned media from a reactor vessel supporting growth of mammalian cells on a microcarrier matrix, said cells being capable of secreting the polypeptides into the conditioned media, wherein said mammalian cells are capable of effecting bead-to-bead transfer without proteolytic enzyme treatment, mechanical disruption, or calcium deprivation;

b) replenishing the reactor vessel with fresh culture media;

c) separating the polypeptides from the recovered conditioned media; and

d) repeating steps a) through c) after a time sufficient for the concentration of polypeptides in the conditioned media to rise to a preselected level.

3. A method as in claim 2, wherein the culture media includes a serum component which has been treated to remove degradative enzymes.

4. A method as in claim 2, wherein the microcarrier bead matrix comprises beads having dimensions in the range from 50 to 300 microns.

5. A method as in claim 2, wherein the polypeptides are tissue plasminogen activator.

6. A method as in claim 2, wherein the cells are from cell line CHL-1.

7. A method for expanding a mammalian microcarrier cell culture within a reactor, said method comprising:

introducing sterile microcarrier beads to the reactor, cell culture already being established within the reactor on a microcarrier bead matrix; and

perfusing culture medium through the microcarrier bead matrix to effect bead-to-bead transfer of the cell culture in the absence of a proteolytic enzyme treatment, mechanical disruption, and calcium deprivation.

8. A method for producing tissue plasminogen activator (t-PA), said method comprising:

perfusing culture media through a microcarrier bead matrix supporting growth of a human melanoma cell line capable of secreting t-PA into the media and effecting bead-to-bead transfer;

collecting conditioned culture medium from the reactor; and

separating t-PA from the collected conditioned culture media.

9. A system for producing a polypeptide, said system comprising:

a reactor vessel;

a microcarrier bead matrix within the reactor vessel;

mammalian cells attached to at least a portion of the microcarrier bead matrix, wherein said mammalian cells are capable of effecting bead-to-bead transfer without proteolytic· enzyme treatment, mechanical disruption, and calcium deprivation;

means for agitating the microcarrier bead matrix under low shear conditions;

means for supplying oxygen to the cells;

means for supplying culture medium to the vessel; and

means for collecting conditioned medium including the polypeptide from the reactor vessel.

10. A composition of matter comprising conditioned media from a mammalian cell line, said conditioned media including tissue plasminogen activator (t-PA) present in its single chain form and as degradation products of the single chain form, wherein the single chain form constitutes at least about 75% of the total weight of the t-PA.

11. A method for producing tissue plasminogen activator (t-PA), said method comprising perfusing culture media through a microcarrier bead matrix supportin growth of a mammalian cell line capable of secreting t-PA into the media under conditions where the secreted t-PA comprises at least about 75% by weight single chain species.

12. A method as in claim 11, wherein the culture media has been treated to remove plasminogen.

ALKALT

SERUM

GAS

10

16

46 48

60

72

22

62

26

58

56

54

30

38 30

70

20

24

32

42

36

12

34

14

FIG._I.

FIG._2.

FIG.__3.

FIG._4.

FIG._5.